# NEW EUROPEAN PATENT SPECIFICATION

(11) **EP 1 874 689 B2**
(45) Date of publication and mention of the opposition decision: **26.10.2016**
(45) Mention of the grant of the patent: 11.12.2013
(21) Application number: 05736193.3
(22) Date of filing: 22.04.2005
(51) Int. Cl.: C01F 5/30, C01B 9/02, A61K 8/20, A61Q 19/00, A61K 33/14, A61K 8/73, A61Q 17/02, A61K 9/00, A61K 47/36, A61K 8/04

(54) **MINERAL SALT GEL COMPOSITIONS**
MINERALSALZGELZUSAMMENSETZUNGEN
COMPOSITIONS EN GEL A BASE DE SELS MINERAUX

(43) Date of publication of application: 09.01.2008
(73) Proprietor: R & H Minerals B.V., 9645 KZ Veendam (NL)
(72) Inventor: HOOIVELD, Lambert, NL-9642 JX Veendam (NL); JANSE, David-Jan, NL-9641 PA Gieten (NL)
(74) Representative: V.O.
(86) International application number: PCT/NL2005/000299
(87) International publication number: WO 2006/112690

(56) References cited:
- EP-A- 1 112 750
- WO-A-99/33443
- WO-A-2004/009101
- WO-A-2004/056334
- US-A- 5 326 432
- US-B1- 6 607 716
- PATENT ABSTRACTS OF JAPAN vol. 1999, no. 06, 31 March 1999 (1999-03-31) & JP 09 291015 A (LG CHEM LTD), 11 November 1997 (1997-11-11)

## Description

The invention relates to salt gel compositions, methods for manufacture and use of the same. In particular, it relates to gel compositions based on MgCl₂.6H₂O (Bischofite) and the cosmetic and therapeutical uses thereof.

Mineral salts are known to have a wide range of therapeutic and cosmetic properties. They can relieve skin ailments such as acne, eczema and psoriasis. Furthermore, they have positive cosmetic effects, like softening, hydrating and inflammation-suppressing effects. Particularly well known is the use of natural salt crystals from the Dead Sea. Dead Sea salts can help strengthen the immune system, detoxify, improve circulation, relieve aches and pains, soften and heal the skin, reduce fatigue, release tension, rejuvenate and revitalize. Bathing in the Dead Sea as treatment of skin diseases has been known for hundreds of years. The Dead Sea has an average salinity of 280 g/kg compared with the ocean's average 35 g/kg. It is rich in magnesium, calcium, potassium, and bromine and is depleted in sodium, sulphate, and carbonate ions. Magnesium salts are quantitatively and qualitatively most important in Dead Sea water. The actual salt content depends on the regional location (water is flowing in by the River Jordan), the water depth, and various additional factors. Salt from the Dead Sea is sold in many countries.

Biochemical effects of magnesium salt therapy have been evidenced *in vitro* and *in vivo*. *In vitro* studies have shown that magnesium bromide and magnesium chloride inhibit the well-known excessive proliferation of psoriatic keratinocytes. Increased levels of magnesium and calcium ions, which may play a role in cell proliferation and differentiation, have been described in psoriatic keratinocytes and after sodium laurylsulfate-induced irritation.

Spasov et al. (Bull Exp Biol Med. 2001;131(2):132-5) reported that local treatment with a Bischofite-containing drug (polykatan™) with up to 95% MgCl₂.6H₂O, promoted healing of infected skin wounds of rats. Washing the wounds with a 10% polykatan solution showed a bacteriostatic effect against a variety of opportunistic micro organisms. Furthermore, the rate of wound healing was enhanced when wounds were treated with the magnesium salt solution as compared to a NaCl solution isotonic to polykatan solution or to untreated controls. Magnesium ions also exhibit anti-inflammatory properties; a magnesium ion-containing ointment significantly inhibited the croton-oil-induced inflammation of the skin. Furthermore, beneficial effects of magnesium ions applied locally to the skin of patients with contact dermatitis have been reported (Greiner J, Diezel W. Entzündungshemmende Wirkung von Magnesium-Ionen bei der Kontaktekzem-Reaktion. Hautarzt 1990; 41: 602-605.12)

Magnesium ions inhibit the antigen-presenting capacity of Langerhans' cells, most important for sensitization and elicitation of allergic reactions; contributing to the efficacy of Dead Sea salt in the treatment of inflammatory skin diseases. At the Dead Sea, bathing in the salt water is usually combined with ultraviolet (UV) irradiation. Although such a combination is effective, the benefit of the bathing aspect has been emphasized by Even-Paz et al.,(Dead Sea sun versus Dead Sea water in the treatment of psoriasis. J Dermatol Treat 1996; 7: 83-86). The authors found improvement in psoriasis patients who only bathed in Dead Sea water. Proksch et al. (Int J Dermatol. 2005; 44(2):151-7) examined the effect of bathing in a Dead Sea salt solution especially rich in magnesium ions on biophysical characteristics in atopic dry skin. It was found that bathing in a magnesium-rich Dead Sea salt solution improves skin barrier function, enhances skin hydration, and reduces inflammation in atopic dry skin.

Another common bath salt is Epsom salt, which is made up of hydrated magnesium sulphate. Epsom salt baths are used for combating stress and alleviating muscular aches and pains. The high magnesium content in Epsom salt baths also facilitates the removal of acids through the skin.

Therapeutic mineral salts are typically provided in the form of crystal-like granular or flaky products. The treatment with a beneficial or therapeutic mineral salt has thus far primarily involved bathing or immersing the affected body or skin parts in an aqueous solution of the salt. A granular form is advantageous for a rapid dissolution in water. The smaller the salt's granule size is, the faster it dissolves in the bath. However, it will be understood that when using dissolved salts, the salt will be significantly diluted and the (skin) surface to be treated is only exposed to minor amounts of the beneficial salt. Salt solutions thus do not allow to apply large amounts of salt per unit of skin surface. As a result, the cosmetic and/or therapeutic effectiveness of salts when used in solution is relatively low. Furthermore, because of the rapid dissolution of granular mineral salts in water, these salts are inefficient and cumbersome to handle for application of and unsuitable for use under the shower. Following application of salt granules onto the body, the salts easily dissolve in the water emerging from the shower. Moreover, a substantial proportion of the granules falls down and is rinsed away before having been in contact with the body.

Therefore, it is an object of the present invention to provide a salt formulation that allows for a convenient application of a therapeutic or cosmetic mineral salt, in particular magnesium salt, in a concentrated form.

Surprisingly, it has been found that the objects set are achieved.by the provision of a salt gel composition comprising MgCl₂.6H₂O in an amount at least 60 percent by weight (w%) and a gelling agent, wherein the gelling agent is present in an amount of at least 0.05% by weight for each 1% of liquid to be gelated in the composition. Such a salt gel is chemically and physically stable for extended periods of time. It allows to expose a body part to a high concentration of the beneficial magnesium salt for a prolonged period of time. Furthermore, the saltgel can be packaged in a tube, container or pump dispenser which is both user-friendly and protects the hygroscopic magnesium salt against excessive moist.

MgCl₂.6H₂O (magnesium chloride hexahydrate) is a salt hydrate that is also known as Bischofite. Bischofite is a crystalline salt hydrate left after evaporation of an ancient sea. Bischofite is are extremely hygroscopic; it melts in the open air. As a mineral, Bischofite is encountered as a glomeroblastic salt rock. Pure Bischofite crystals are aquatic-transparent, but may also be of white, rose and fallow colour depending on impurities. Bischofite is encountered on many continents, in formations differing in age including modern ones. There were discoveries of small deposits in Western Ukraine, Byelorussia and Kazakhstan. All the discoveries were represented by thin (3 to 7 m) non-extended layers. Most notable potassium and magnesium salt deposits are found in Carlsbad, New Mexico; the Paradox Basin in Colorado and Utah; deposits in Strassfurt, Germany; the Perm Basin, Russia and the Williston Basin in Saskatchewan, Canada. Another natural source of Bischofite is the region around Veendam, The Netherlands. NEDMAG INDUSTRIES Mining and Manufacturing B.V. mines the Bischofite salt from the Zechstein salts near Veendam with a unique solution mining method.

Whereas a gel comprising 60% of the magnesium salt is sufficiently concentrated to obtain satisfactory cosmetic or therapeutic effects, a higher salt concentration is preferred. Particularly good results are obtained using a composition comprising at least 70w%, more preferably at least 80w% MgCl₂.6H₂O. For example, the invention provides a stable salt gel composition which contains 85 w% MgCl₂.6H₂O.

The gelling agent that is used to formulate a viscous Bischofite gel composition is preferably present in an amount of at least 0.5% by weight, more preferably at least 1 w% based on the Bischofite content of the composition. For example, a composition of the invention comprises around 60% Bischofite and 3% gelling agent, both percentages being expressed to the total weight of the salt gel composition. In another embodiment, around 1w% gelling agent is used relative to the Bischofite content, for example in a concentrated salt gel composition with 85% Bischofite and 1% gelling agent. The higher the salt content, the less gelling agent will be required to achieve the desired stabilized salt concentrate, because less free water is present. As a general rule of thumb, approximately 0.06% of gelling agent is required to prepare a stable gel composition from a composition with 1% (by volume) of "free" water. For example, a brine with 64% MgCl₂.H₂O (30% MgCl₂) contains 46% water. Accordingly, approximately 2.8-3.0% of gelling agent should be used. Likewise, a 85% Bischofite brine with only 15% free water only requires around 0.9-1.0% of gelling agent. Thus, according to the invention the gelling agent is present in the composition in an amount of at least around 0.06% for each 1% of liquid present in the composition to be gelated. The amount of gelling agent to be used will however not only depend on the salt content of the composition, but also on other factors, like the type of gelling agent and the desired viscosity. For example, the gelling agent is present in an amount of 0.1%, 0.2%, 0.4%, 1%, 2%, 3% or more, for example 5%. The gelling agent is preferably present in amount of up to 8-10% for each percentage of liquid to be gelated, although this may depend on the type of gelator used. At a gelling agent concentration of more than approximately 0.3 w% for each percentage of free water , a highly viscous composition can be obtained which has a pad-like, semi-transparent appearance. In one embodiment, a 80% Bischofite composition is prepared using at least 6% gelling agent. The term 'salt gel composition' as used herein is thus not limited to compositions with semi-solid, paste-like properties but also encompasses gel pads.

Several types of gelling agents (sometimes also referred to as binders) can be used to prepare a salt gel composition. Natural, i.e. non-toxic, gelling agents are of course preferred if the composition is to be used as cosmetic or therapeutic composition. Especially at high concentrations of salt, the use of non-ionic gelling agents is preferred as these are only minimally affected by salts. Examples of useful gelling agents to formulate a Bischofite gel are starch and cellulose derivatives, for example hydroxyethyl derivatives, hydroxypropylmethyl derivatives and hydroxypropyl derivatives. Hydroxypropyl derivatives were found to be particularly useful.

In one embodiment, the gelling agent is hydroxypropyl cellulose (HPC). In another embodiment, use is made of hydroxypropyl starch, preferably in an amount of 1-5% by weight based on the salt hydrate. Hydroxypropyl starch is a derivative of natural starch, used primarily for fluid-loss control in drilling muds, drill-in, completion and workover fluids. Being non-ionic, it is only slightly affected by salinity and hardness in fluids. Linear and branched carbohydrate polymers in natural starch have three reactive OH groups on each glucose unit. During manufacture, these polymers are reacted with propylene oxide, adding hydroxypropyl (CH(OH)CH₂CH₃) groups at the OH positions by an ether linkage.

Another preferred gelling agent is hydroxypropyl starch phosphate, also known under the trade name Structure™ XL. Structure XL is commercially available from National Starch and Chemical Company, a member of the ICI Group. It has been used in personal care emulsions, were it can aid in emulsion stabilization, aesthetics enhancement and viscosity build. An emulsion containing STRUCTURE XL has a good stability over a broad temperature range (-30°C up to 50°C). It also makes the formulation easy to apply to the skin. STRUCTURE XL is readily cold water dispersible so that no pre-mixes are needed. Because of its nonionic character and broad compatibility, STRUCTURE XL allows to formulate over a wide pH range with high amounts of salts and a large variety of raw materials. According to the supplier, STRUCTURE XL is stable in the presence of up to 20% of mono- and divalent salts. The recommended concentrations according to the supplier to improve emulsion stability range from about 3% to 7.5%, while significant viscosity effects were reportedly observed at concentrations > 7.5%. The present inventors surprisingly observed that STRUCTURE XL can be used to formulate a stable gel comprising much higher concentrations of Bischofite; 1w% STRUCTURE XL was capable of stabilizing a salt gel comprising 80w% MgCl₂.6H₂O.

A concentrated magnesium chloride gel of the invention can be used for several purposes, ranging from personal care use (such as cosmetic or therapeutic use) to industrial and agricultural applications. For example, the invention also encompasses the use of a Bischofite gel as dust absorber or dust suppressor. Due to the presence of the gelling agent in the salt composition, the suppressive action is improved and better controllable. The gelling agent somewhat reduces the hygroscopic action of the Bischofite (the salt is surrounded by the agent) which results in a prolonged dust suppressing effect. Also, the gelling agent 'glues' the Bischofite to the sand or dust particles. In another aspect, a mineral salt gel composition as provided herein finds its use in animal feed. The gel is more easily mixed with other components of the animal feed compared to existing sources of MgCl₂, e.g. pure Bischofite. In addition, if incorporated in the food as a gel, the bitter taste of pure Bischofite is somewhat masked
In every application, the concentrated salt gel forms the basis. Depending on the application one or more additional ingredients can of course be present in the salt gel. In one embodiment, the salt gel is a cosmetic composition. The presence of a high concentration magnesium chloride hexahydrate can have beneficial effects on the skin. The salt gel composition may further comprise other substances that contribute to the attractiveness and/or cosmetic properties of the composition. Examples of substances that may be added to the salt composition include fragrances, e.g. etheric oils, pigments, plant extracts like tea tree, neem, aloe vera, calendula, jojoba and the like. In a particular aspect, the invention provides a mineral scrub gel on the basis of Bischofite. To that end, an exfoliating substance is included in the gel, for instance coarse sea salt crystals, sugar, ground apricot- or cherry pits or crushed olive pits or a salt with known therapeutical and/or cosmetic properties, for example (Dead) sea salt crystals. Useful exfoliating salt additives include particles of those salts which do not dissolve in the mineral gel of the invention, for instance sodium chloride, carnallite and magnesium sulphate. A mineral scrub gel may for example comprise 70- 85% Bischofite and 1-2.5% of the gelling agent Structure XL.

Another aspect of the present invention relates to the treatment of ecto-parasitic infestations, in particular lice (pediculosis). These infestations are a common problem among children. They are often spread from shared hats, combs, lockers, closets and other unavoidable contacts in school. Infestations can easily spread to other children and family members by touching or via shared clothing. Conventional approaches to combat pediculosis typically involve the use of chemical insecticides, such as DDT, hexachlorocyclohexane (HCH), malathion, pyrethrins, permethrins, and the like. Not only is the use of these toxic and unpleasant chemical insecticides highly undesirable, they also induce resistance in the target pest against the insecticides. Lice resistant to DDT and HCH have been observed in Canada, the USA and Europe.

The present inventors surprisingly observed that a concentrated Bischofite gel is advantageously used to treat infestation of lice fleas and similar ectoparasites. The salt composition of the invention kills target pests by virtue of its strong hygroscopic properties. Of course, this mode of killing is very unlikely to induce resistance. Furthermore, the active ingredient is not toxic. Rather, the salt gel composition can be conveniently applied to the scalp (or any other infested area) for prolonged periods without causing any harm or irritation. The salt gel can be massaged into the hair to thoroughly coat the hair. The gel formulation sticks to the hair and avoids excessive dripping or spilling, even during prolonged periods of time. Subsequently, a plastic cap can be put over the head. After about an hour, the maximum amount of time it typically takes to kill the ecto-parasites, the salt paste can be simply rinsed off the hair and scalp with water. The dead parasites and, if present, their eggs can be combed out using a special comb. It was found that this treatment is more than 90% effective, not only against lice but also against nits and unhatched eggs in every stage of development. The salt has a softening and smoothening effect on the hair, thereby further enhancing the detachment of nites from the hair. Furthermore, minor infections or small wounds which may be present in the treated area will be exposed to the beneficial effects of Bischofite.

Thus, a salt gel composition of the invention is advantageously used to combat ecto-parasites, i.e. parasites which live on the outer surface of its host. The treatment may be repeated after some time (e.g. 7-10 days) following the first treatment. The host can be any organism suffering from an ecto-parasite infestation, including human beings and cattle. The major cattle ectoparasites are ticks and biting flies. Their bites cause irritations, preventing the cattle from grazing, and can also lead to secondary infections and infestations. As bloodsuckers, these parasites will also cause anaemia when present in high numbers, weakening the animals and even killing younger ones. Even more important, several of these bloodsucking parasites also act as vectors, meaning that they transmit various very debilitating or lethal diseases to their host.

The content of the mineral salt and gelling agent in an anti-parasitic gel composition according to the invention can vary, depending on several factors such as the mode of application, the type of gelling agent, the subject to be treated (human or animal) and the ectoparasite to be killed. In one embodiment, it comprises at least 60% Bischofite, preferably at least 70% Bischofite. For example, provided is an anti-parasitic salt gel composition comprising around 85% Bischofite and 1.5% of a hydroxypropyl starch gelling agent (e.g. Structure XL). In another embodiment, a mineral salt gel composition is provided which is less viscous (e.g. comprising 70-85% Bischofite and around 1% gelling agent) and allows application to the subject to be treated by way of spraying. This form of application is particularly advantageous for the treatment of cattle.

Pediculicidal compositions comprising salts have been described in US 6,607,716. The suggested amounts of effective salt range from 1 to 50 weight percent, with a preferred range of 5 to 20 w%, more preferred 10w%. The preferred salt in US 6,607,716 is sodium chloride. Concentrated salt compositions with at least 60w% MgCl₂.6H₂O as provided herein were not disclosed.
Still a further aspect of the invention relates to the therapeutic use of salt gel pads of the invention. As mentioned above, magnesium salts have anti-inflammatory and bacteriostatic effects and stimulate the rapid natural closure of wounds. The moist salt pad keeps wound or scars moist and soft, while the magnesium chloride can exert its wide range of beneficial effects. A flexible salt gel pad comprising magnesium chloride of the invention is thus advantageously used as dressing for wounds, including chronic wounds and burn wounds, and for the local treatment of symptoms associated with (inflammatory) skin disorders, such as psoriasis. A salt gel pad may also be used in the area of plastic and reconstructive surgery. The salt gel pad is preferably contacted directly with the affected area. If a prolonged exposure to the salt is desired, it is practical to immobilize the pad. This can be done with a plaster, bandage, or the like. The plaster or bandage may be used as a separate article or it may be an integral part of the pad. The latter can be achieved during the production of the pad, by contacting the salt gel composition while still in a liquid form with a plaster, bandage or the like. The size and shape of the pad can vary. It will generally have a thickness of between 0.3 and 2 centimetre. The salt gel not only has beneficial effects on its own based on the magnesium salt but it also provides an excellent matrix for the release of other therapeutic or cosmetic substances. Thus, the gel pad may also comprise additional ingredients that are of benefit to the user, for example additional anti-inflammatory or anti-microbial compounds, and/or factors which enhance wound healing, for instance aloe vera.

A mineral gel composition of the invention is typically prepared in two main stages. In the first stage, a salt concentrate is obtained. During the second stage, the salt concentrate is stabilized with a gelling agent. The salt concentrate can be prepared using either a solid or liquid starting material, or a mixture thereof.

The solid starting material can be salt flakes comprising MgCl₂.6H₂O, for example Bischofite salt flakes. In one embodiment, water and salt flakes are combined in a container and the mixture is heated while stirring until all the salt flakes are dissolved. In one embodiment, a mixture of 85% Bischofite in is heated to about 109°C. Mixtures with a lower or higher Bischofite concentration may be heated to other temperatures to cause melting of the salt flakes. These temperatures can be easily determined imperically. Heating of the salt/water mixture can be performed by any means, for instance electrically, using steam or using hot oil. Thereafter, the mixture is allowed to cool to a temperature below 40 °C under constant stirring. The container can be placed in a bath with cold water. The stirring ensures that the crystals which form at the walls of the container upon cooling are scraped off the wall, allowing new crystals to form at the cold wall. Also, this avoids the formation of large salt crystals such that the final gel composition has a fine structure.

The liquid starting material for preparing a salt concentrate can be a salt melt or a mixture of a salt solution to which a salt melt is added. In one embodiment, a solution of Bischofite at a known concentration, for example prepared from flakes or from a concentrated brine, is brought in a stirred container, preferably a double-walled metal container which can be actively cooled. A Bischofite melt, prepared by heating Bischofite to a temperature at which a homogenous melt is obtained which contains no solid Bischofite, is added to the solution in a controlled fashion while stirring and cooling the mixture. The rate at which the melt is added to the solution should be low enough to avoid boiling of the mixture and high enough to prevent too fast crystallization to occur. In this respect, the use of a salt solution with at least 60w%, preferably at least 65% Bischofite is preferred because this ensures a controlled and efficient mixing with the added melt while avoiding that the mixture comes to a boil.
In a variant of the above method, it is also possible to start with an aqueous salt solution which is heated in a controlled manner to evaporate excess water and to prepare a salt melt with a desired salt content. In one embodiment, said aqueous solution is a brine of crude Bischofite which is obtained from the earth using solution mining. In solution mining, water is pumped into the underground salt cavern to extract salt. Here, the salt mixes with the water to form a brine solution. This solution is then easily pumped to the surface. The brine solution is heated in an evaporator to evaporate water and to obtain a concentrated (e.g. 85 w%) Bischofite melt. The salt melt is then transferred to a crystallizer (e.g. a stirred double-walled metal container) where it is cooled down to form a crystalline salt concentrate. This crystalline salt concentrate is ready for further mixing with gelling agent. This variant method is particularly attractive for large scale preparations, for example starting from freshly mined Bischofite brine.

Following the preparation of a salt concentrate using cooling and crystallization to < 40°C, a gelling agent is added while stirring. The gelling agent is preferably added to the salt concentrate as a powder. To obtain a stable salt gel composition, it is preferred that the salt/ gelling agent mixture is allowed to 'mature" for at least several hours, e.g. 12 hours or even more. This ensures that the gelling agent has come into maximal contact with the free water molecules. For instance, if a starch-based gelling agent is used a maximal swelling of the starch moieties is achieved during the maturation period. Following the maturation period, the composition is generally stirred again to obtain a homogenous and stable salt gel composition. It this point in the procedure one or more additives (see above) may be added to the composition. Herewith, the invention provides a method for preparing a salt gel composition according to any of the previous claims, comprising the steps of: a) providing a starting composition comprising MgCl₂.6H₂O in a dissolved and/or molten form; b) cooling said starting composition to a temperature at which the gelling agent has adequate gelling properties while stirring to obtain a salt concentrate comprising the desired amount of MgCl₂.6H₂O; c) mixing said salt concentrate with a suitable amount of gelling agent; d) allowing the mixture obtained in step c) to mature; and e) homogenizing the matured mixture obtained in step d), optionally while adding one or more further ingredients. The temperature at which the gelling agents has sufficient gelling properties depends of various factors, in particular the type of gelling agent use. For example, the gelling agent Structure XL can be used as a gelling agent in the range of -30°C to 50°C. In that case, the mixture is preferably cooled to a temperature below 50°C, such as 40°C or lower. For other types of gelling agents, other temperatures may be suitable. These can be empirically determined by a person skilled in the art.
The salt gel composition is ready for packaging in the desired package material. Various types of packaging materials may be used. Preferably, the packaging material is provided with a resealable closure. This is user-friendly and also prevents unwanted moist access to the strongly hygroscopic salt concentrate. Examples of useful packaging material for paste-like salt gel compositions include a tube (e.g. 100-500 ml) with a screw cap or click-on cap. The tube is preferably made of a flexible plastic. Other examples are containers of any sort or shape with a screw cap or click-on cap. For large scale applications, packaging materials with a dispenser system are preferred. The gel pads can be sealed in a moisture resistant, e.g. plastic, wrapping.

Furthermore, the invention provides a kit for the treatment of an ecto-parasite infestation comprising a Bischofite-based salt gel composition according to the invention. The composition can be present in any type of container or tube. A kit of the invention is for example a kit for the treatment of a lice infestation. The kit may contain additional useful items, such as a plastic hair cap, a comb and/or instructions for use.

The invention is illustrated by the Examples below.

### Example 1: MINERAL GEL for disinfection of the skin comprising 65% Bischofite and 3% Structure XL as gelling agent.

This form of the mineral gel is comparable with conventional disinfecting soap gels in terms of viscosity and application range. However, in the mineral salt gel the disinfecting properties can be ascribed to the Bischofite. Washing the skin (e.g. hands) with the salt gel of relatively low viscosity can disinfect the skin. Following treatment with the salt gel, it can be easily rinsed off the skin with water. The mineral salt gel may also comprise a fragrance and pigment to increase the attractiveness of the gel.

### Example 2: MINERAL GEL for the treatment of skin disorders comprising 85% Bischofite and 1 à 2 % Structure XL

Mineral salts in this type of gel formulation allow for a very convenient application of the salt to the skin. The mineral can be applied as a gel packing. The gel may be massaged lightly onto the skin. The amount of gel to be used can vary. Generally speaking, a layer of several millimetres will be sufficient to exert the beneficial therapeutic effects of the Bischofite. The mineral gel has no scrub-like properties exfoliating the skin but can soak off dead skin cells. Thereby, the skin becomes more susceptible to the caring, healing and clearing properties of Bischofite.

This type of gel is suitably used for the treatment of acne. The Bischofite mineral cleanses the infected skin. dehydrates and drains the pores, thus preventing new pimples from forming. Possible scars due to the inflammation will heal more efficiently.

In case a long-term treatment of skin with a mineral salt gel is desired, the salt gel may be covered with Saran-wrap following application onto the skin.

### Example 3 : MINERAL PAD (gel pad) comprising 85% Bischofite and 3-5% Structure XL.

In the mineral pad, the minerals are formulated into a thick, highly viscous dosage form which sticks to the site of application. Following application of the pad to the desired site it may be pressed onto the skin into a thinner pad which sticks for even longer periods of time. The pad may be covered with a bandage or plaster, e.g. to allow for more freedom to move while the minerals can exert their effects on the skin. The gel pad is suitably used for the treatments of small wounds or to combat acne. The mineral salts can be easily rinsed off with water after completion of the treatment.

## Claims

1. A salt gel composition comprising at least 60% by weight of MgCl₂.6H₂O (Bischofite) and a gelling agent, wherein the gelling agent is present in an amount of at least 0.05% by weight for each 1% of liquid to be gelated in the composition.

2. A salt gel composition according to claim 1, wherein said gelling agent is a non-ionic derivative of starch or cellulose.

3. A salt gel composition according to claim 2, wherein the gelling agent is a hydroxypropyl starch.

4. A salt gel composition according to claim 3, wherein the gelling agent is hydroxypropyl starch phosphate (Structure™ XL).

5. A salt gel composition according to any of the previous claims, further comprising at least one additive, preferably a cosmetic or therapeutic additive.

6. A salt gel composition according to claim 5, wherein said additive is selected from group consisting of an exfoliating substance, a fragrance, a pigment, a salt, an anti-microbial substance, an anti-inflammatory substance, a plant extract, foaming agent and an oil, such as an etheric oil.

7. A salt gel composition according to any of the previous claims, wherein the gelling agent is present in an amount of at least 0.3% by weight for each 1% of liquid to be gelated in the composition, such that the composition has the form of a gel pad.

8. Method for preparing a salt gel composition according to any of the previous claims, comprising the steps of:
a) providing a starting composition comprising MgCl₂.6H₂O in a dissolved and/or molten form;
b) cooling said starting composition to a temperature at which the gelling agent displays gelling properties while stirring to obtain a salt concentrate comprising the desired amount of MgCl₂.6H₂O;
c) mixing said salt concentrate with a suitable amount of gelling agent;
d) allowing the mixture obtained in step c) to mature; and
e) homogenizing the matured mixture obtained in step d), optionally while adding one or more further ingredients.

9. Method for preparing a salt gel composition in the form of a gel pad, comprising the method of claim 8, wherein the gelling agent is added to the salt concentrate in an amount of at least 0.3% by weight for each 1% of liquid to be gelated in the concentrate.

10. Use of a salt gel composition according to any one of claims 1 to 7 as a cosmetic substance.

11. Salt gel composition according to any one of claims 1 to 7, for use as a dermatological composition.

12. Salt gel composition according to any one of claims 1 to 7, for use in a method of treatment of an inflammatory disease.

13. Salt gel composition according to any one of claims 1 to 7, for use in a method of treatment of wounds..

14. Salt gel composition according to any one of claims 1 to 7, for use in a method of treatment of an ecto-parasite infestation in an animal, preferably a human.

15. Salt gel composition according to claim 14, wherein said ectoparasite infestation is a lice infestation.

16. Kit for the treatment of an ecto-parasite infestation comprising a salt gel composition according to any one of claims 1 to 7.

17. Kit according to claim 16 for the treatment of a lice infestation, further comprising a plastic hair cap, a comb and/or instructions for use.

## Patentansprüche

1. Salzgelzusammensetzung, umfassend mindestens 60 Gewichtsprozent MgCl₂6H₂O (Bischofit) und ein Geliermittel, wobei das Geliermittel in einer Menge von mindestens 0,05 Gewichtsprozent pro 1 % an Flüssigkeit, die in der Zusammensetzung geliert werden soll, vorhanden ist.

2. Salzgelzusammensetzung nach Anspruch 1, wobei das Geliermittel ein nichtionisches Derivat von Stärke oder Zellulose ist.

3. Salzgelzusammensetzung nach Anspruch 2, wobei das Geliermittel eine Hydroxypropylstärke ist.

4. Salzgelzusammensetzung nach Anspruch 3, wobei das Geliermittel Hydroxypropylstärkephosphat (Structure™ XL) ist.

5. Salzgelzusammensetzung nach einem der vorhergehenden Ansprüche, ferner umfassend mindestens ein Additiv, vorzugsweise ein kosmetisches oder therapeutisches Additiv.

6. Salzgelzusammensetzung nach Anspruch 5, wobei das Additiv ausgewählt ist aus einer Gruppe bestehend aus einer abschälenden Substanz, einem Duft, einem Pigment, einem Salz, einer antimikrobiellen Substanz, einer entzündungshemmenden Substanz, einem Pflanzenextrakt, einem Schäumungsmittel und einem Öl wie beispielsweise einem ätherischen Öl.

7. Salzgelzusammensetzung nach einem der vorhergehenden Ansprüche, wobei das Geliermittel in einer Menge von mindestens 0,3 Gewichtsprozent pro 1 % an Flüssigkeit, die in der Zusammensetzung geliert werden soll, vorhanden ist, so dass die Zusammensetzung die Form eines Gelpads aufweist.

8. Verfahren zur Herstellung einer Salzgelzusammensetzung nach einem der vorhergehenden Ansprüche, umfassend folgende Schritte:
a) Bereitstellen einer Startzusammensetzung, umfassend MgCl₂.6H₂O in gelöster und/oder geschmolzener Form;
b) Abkühlen der Startzusammensetzung auf eine Temperatur, bei der das Geliermittel unter Rühren Geliereigenschaften entfaltet, um ein Salzkonzentrat zu erhalten, das die gewünschte Menge an MgCl₂.6H₂O aufweist;
c) Mischen des Salzkonzentrats mit einer geeigneten Menge an Geliermittel;
d) Erlauben des in Schritt c) erhaltenen Gemischs zu reifen; und
e) Homogenisieren des in Schritt d) erhaltenen gereiften Gemischs, optional unter Hinzufügung von einer oder mehreren weiteren Zutaten.

9. Verfahren zur Herstellung einer Salzgelzusammensetzung in Form eines Gelpads, umfassend das Verfahren nach Anspruch 8, wobei das Geliermittel dem Salzkonzentrat in einer Menge von mindestens 0,3 Gewichtsprozent pro 1 % an Flüssigkeit, die in dem Konzentrat geliert werden soll, hinzugefügt wird.

10. Verwendung einer Salzgelzusammensetzung nach einem der Ansprüche 1 bis 7 als eine kosmetische Substanz.

11. Salzgelzusammensetzung nach einem der Ansprüche 1 bis 7 zur Verwendung als eine dermatologische Zusammensetzung.

12. Salzgelzusammensetzung nach einem der Ansprüche 1 bis 7 zur Verwendung in einem Verfahren zur Behandlung einer Entzündungskrankheit.

13. Salzgelzusammensetzung nach einem der Ansprüche 1 bis 7 zur Verwendung in einem Verfahren zur Behandlung von Wunden.

14. Salzgelzusammensetzung nach einem der Ansprüche 1 bis 7 zur Verwendung in einem Verfahren zur Behandlung eines Ektoparasitenbefalls bei einem Säuger, vorzugsweise einem Menschen.

15. Salzgelzusammensetzung nach Anspruch 14, wobei es sich bei dem Ektoparasitenbefall um einen Läusebefall handelt.

16. Kit zur Behandlung eines Ektoparasitenbefalls, umfassend eine Salzgelzusammensetzung nach einem der Ansprüche 1 bis 7.

17. Kit nach Anspruch 16 zur Behandlung eines Läusebefalls, ferner umfassend eine Haarkappe aus Plastik, einen Kamm und/oder Gebrauchsanweisungen.

## Revendications

1. Composition de gel à base de sel, comprenant au moins 60 % en poids de bischofite, de formule MgCl_{2·}6H₂O, et un agent gélifiant, dans laquelle l'agent gélifiant se trouve présent en une proportion d'au moins 0,05 % en poids, pour chaque pour-cent de liquide à gélifier présent dans la composition.

2. Composition de gel à base de sel, conforme à la revendication 1, dans laquelle ledit agent gélifiant est un dérivé non-ionique d'amidon ou de cellulose.

3. Composition de gel à base de sel, conforme à la revendication 2, dans laquelle ledit agent gélifiant est un hydroxypropyl-amidon.

4. Composition de gel à base de sel, conforme à la revendication 3, dans laquelle ledit agent gélifiant est un phosphate d'hydroxypropyl-amidon (Structure^{®} XL).

5. Composition de gel à base de sel, conforme à l'une des revendications précédentes, qui comprend en outre au moins un adjuvant, de préférence un adjuvant cosmétique ou thérapeutique.

6. Composition de gel à base de sel, conforme à la revendication 5, dans laquelle ledit adjuvant est choisi dans l'ensemble formé par une substance exfoliante, un parfum, un pigment, un sel, une substance anti-microbienne, une substance anti-inflammatoire, un extrait de végétal, un agent moussant, et une huile, telle une huile essentielle.

7. Composition de gel à base de sel, conforme à l'une des revendications précédentes, dans laquelle l'agent gélifiant se trouve présent en une proportion d'au moins 0,3 % en poids, pour chaque pour-cent de liquide à gélifier présent dans la composition, si bien que la composition présente l'aspect d'un bloc de gel.

8. Procédé de préparation d'une composition de gel à base de sel, conforme à l'une des revendications précédentes, comportant les étapes suivantes :
a) prendre une composition de départ comprenant du sel de formule MgCl₂·6H₂O à l'état dissous et/ou fondu ;
b) refroidir cette composition de départ, tout en l'agitant, jusqu'à une température à laquelle l'agent gélifiant fait preuve de propriétés gélifiantes, pour obtenir une solution concentrée de sel qui contient la quantité voulue de sel de formule MgCl₂·6H₂O;
c) mélanger cette solution concentrée de sel avec une quantité appropriée d'agent gélifiant ;
d) laisser mûrir le mélange obtenu lors de l'étape (c) ;
e) et homogénéiser le mélange mûri obtenu lors de l'étape (d), tout en y ajoutant, en option, un ou plusieurs autre(s) ingrédient(s).

9. Procédé de préparation d'une composition de gel à base de sel sous forme de bloc de gel, comportant un procédé, conforme à la revendication 8, dans lequel on ajoute l'agent gélifiant à la solution concentrée de sel en une proportion d'au moins 0,3 % en poids, pour chaque pour-cent de liquide à gélifier présent dans la solution concentrée.

10. Utilisation d'une composition de gel à base de sel, conforme à l'une des revendications 1 à 7, en tant que substance cosmétique.

11. Composition de gel à base de sel, conforme à l'une des revendications 1 à 7, pour utilisation en tant que composition dermatologique.

12. Composition de gel à base de sel, conforme à l'une des revendications 1 à 7, pour utilisation dans un procédé de traitement d'une maladie inflammatoire.

13. Composition de gel à base de sel, conforme à l'une des revendications 1 à 7, pour utilisation dans un procédé de traitement de plaies.

14. Composition de gel à base de sel, conforme à l'une des revendications 1 à 7, pour utilisation dans un procédé de traitement d'une infestation par un ectoparasite chez un animal, de préférence chez un humain.

15. Composition de gel à base de sel, conforme à la revendication 14, pour laquelle l'infestation par un ectoparasite est une infestation par des poux.

16. Trousse pour le traitement d'une infestation par un ectoparasite, comprenant une composition de gel à base de sel, conforme à l'une des revendications 1 à 7.

17. Trousse, conforme à la revendication 16, pour le traitement d'une infestation par des poux, comprenant en outre un couvre-cheveux en plastique, un peigne et/ou un mode d'emploi.
